(19) Europäisches Patentamt
European Patent Office
Office européen des brevets

(11) **EP 2 957 289 A1**

(12) **EUROPEAN PATENT APPLICATION**
published in accordance with Art. 153(4) EPC

(43) Date of publication:
**23.12.2015 Bulletin 2015/52**

(21) Application number: **14751155.4**

(22) Date of filing: **14.02.2014**

(51) Int Cl.:
**A61K 36/00** (2006.01)   **A61K 36/18** (2006.01)
**A61K 36/28** (2006.01)   **A61K 36/53** (2006.01)
**A61P 9/10** (2006.01)   **A61P 19/02** (2006.01)
**A61P 19/10** (2006.01)   **A61P 25/28** (2006.01)
**A61P 27/12** (2006.01)   **A61P 29/00** (2006.01)
**A61P 43/00** (2006.01)

(86) International application number:
**PCT/JP2014/053474**

(87) International publication number:
**WO 2014/126199 (21.08.2014 Gazette 2014/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **18.02.2013 JP 2013029339**

(71) Applicants:
• **ARKRAY, Inc.
Minami-ku
Kyoto-shi
Kyoto 601-8045 (JP)**
• **The Doshisha
Kyoto-shi, Kyoto 602-8580 (JP)**

(72) Inventors:
• **YAGI Masayuki
Kyotanabe-shi
Kyoto 610-0394 (JP)**
• **YONEI Yoshikazu
Kyotanabe-shi
Kyoto 610-0394 (JP)**
• **TAKAYAMA Kiyofumi
Kyoto-shi
Kyoto 602-0008 (JP)**
• **KAWAI Hiroshige
Kyoto-shi
Kyoto 602-0008 (JP)**

(74) Representative: **Hoffmann Eitle
Patent- und Rechtsanwälte PartmbB
Arabellastraße 30
81925 München (DE)**

(54) **OXIDIZED PROTEIN HYDROLASE ACTIVITY ENHANCING AGENT**

(57)    The present invention provides a novel oxidized protein hydrolase activity enhancing agent. The oxidized protein hydrolase activity enhancing agent of the present invention contains at least one kind of plant extract obtained from a plant selected from the group consisting of water chestnut, stevia, rosemary, thyme, chrysanthemum, savory, mugwort, chestnut, spearmint, marjoram, peppermint, lemon balm, allspice, perilla, basil, and caraway.

EP 2 957 289 A1

**Description**

Technical Field

The present invention relates to an enhancing agent for enhancing the activity of oxidized protein hydrolase.

Background Art

It is known that oxidation reactions and glycation reactions in vivo adversely affect cells and tissues. In particular, the production and accumulation of proteins having undergone an oxidation reaction or a glycation reaction, e.g.,, oxidized proteins or glycated proteins, cause diseases such as diabetes complications, Alzheimer's disease, cataracts, and arteriosclerosis, as well as senescence and hypofunction of tissues such as skin (Non-Patent Document 1). On this account, the oxidized proteins and glycated proteins are called damaged proteins. In particular, accumulation of AGEs (Advanced Glycation End-products), which are end products produced by a non-enzymatic addition reaction of a carbohydrate to a protein, is seen as a problem.

These damaged proteins normally are degraded and removed by proteases such as proteasomes and oxidized protein hydrolase (hereinafter referred to as "OPH"). However, the activities of these enzymes are reduced with age (Non-Patent Document 2). Thus, expectations are rising regarding the possibility of treating and preventing the diseases, the senescence of tissues, and the hypofunction of tissues by enhancing the activities of these enzymes.

Proteasomes are large enzyme complexes that degrade proteins, with examples thereof including 26S proteasome and 20S proteasome. Known as substances that activate proteasomes are soybean saponin, kale extract, silybin, silane compounds, iris extract, and the like (Patent Documents 1 to 5). OPH is a serine protease. As substances that activate OPH, Roman chamomile (*Anthemis nobilis L.*), chameleon plant (*Houttuynia cordata Thunberg*), whitethorn (*Crataegus oxyacantha L.*), grapes (*Vitis vinifera L.*), and the like are known (Patent Document 6). However, in recent years, there are increasing demands for a novel, more potent activity enhancing agent.

Citation List

Patent Document(s)

Patent Document 1: JP 2002-179592 A
Patent Document 2: Japanese Patent No. 4255432
Patent Document 3: JP 2008-308505 A
Patent Document 4: JP 2004-91398 A
Patent Document 5: JP 2007-99650 A
Patent Document 6: WO 2011/004733

Non-Patent Document(s)

Non-Patent Document 1: Goto et al., Experimental Medicine, 18 (special number), pp. 2324-2331, 1998
Non-Patent Document 2: Breusing N. et al., Biol. Chem., Vol. 389, pp. 203-209 (2008)

Problem to Be Solved by the Invention

With the foregoing in mind, it is an object of the present invention to provide a novel OPH activity enhancing agent.

Means for Solving Problem

The present invention provides an OPH activity enhancing agent containing: at least one kind of plant extract obtained from a plant selected from the group consisting of water chestnut, stevia, rosemary, thyme, chrysanthemum, savory, mugwort, chestnut, spearmint, marjoram, peppermint, lemon balm, allspice, perilla, basil, and caraway.

The present invention also provides a method for enhancing OPH activity, including the step of adding the OPH activity enhancing agent of the present invention to a sample containing OPH.

The present invention also provides a method for accelerating degradation of a protein, including the step of:

adding the OPH activity enhancing agent of the present invention to a sample containing a protein and OPH, wherein the protein includes at least one of an oxidized protein and a glycated protein.

The present invention also provides a method for treating diseases, senescence of tissues, and hypofunction of tissues caused by production of an oxidized protein or a glycated protein, including administering the OPH activity enhancing agent of the present invention to a subject (living organism).

Effects of the Invention

According to the present invention, it is possible to enhance OPH activity. Thus, the present invention can accelerate the degradation of damaged proteins, e.g., oxidized proteins and glycated proteins such as AGE-modified proteins. Therefore, the present invention can treat and prevent diseases, senescence of tissues, and hypofunction of tissues caused by the damaged proteins produced in vivo, for example. Furthermore, the present invention is excellent in terms of safety because it uses plant extracts that have been used since ancient times. Moreover, the present invention is excellent in terms of productivity because the plant extracts can be obtained in large quantities.

Mode for Carrying out the Invention

In the present invention, the enhancement of the activity of an enzyme may be either activation of the enzyme or inhibition of deactivation of the enzyme, for example. In the present invention, OPH also is referred to as acylamino-acid-releasing enzyme (AARE) or acylpeptide hydrolase (ACPH).

As described above, the OPH activity enhancing agent according to the present invention contains at least one kind of plant extract obtained from a plant selected from the group consisting of water chestnut, stevia, rosemary, thyme, chrysanthemum, savory, mugwort, chestnut, spearmint, marjoram, peppermint, lemon balm, allspice, perilla, basil, and caraway. The OPH activity enhancing agent may contain an extract of one of the above-described plants, extracts of two or more of the above-described plants, or extracts of all of the above-described plants, for example.

The OPH activity enhancing agent of the present invention can enhance the OPH activity. Thus, according to OPH activity enhancing agent of the present invention, it is possible to degrade an oxidized protein or a glycated protein such as an AGE-modified protein efficiently. The OPH activity enhancing agent of the present invention can degrade an AGE-modified albumin or an AGE-modified collagen efficiently through the enhancement of the OPH activity, for example. Albumin generally is known as a protein present widely over the whole body. Thus, because the OPH activity enhancing agent of the present invention can degrade the AGE-modified albumin efficiently, the OPH activity enhancing agent can bring about a systemic effect, for example, and as a specific example, it can be said that the OPH activity enhancing agent is useful in improvement of metabolism. Collagen generally is known as a protein present in skin, tendons, bones, cartilage, and the like. Thus, because the OPH activity enhancing agent of the present invention can degrade the AGE-modified collagen efficiently, it can be said that, for example, the OPH activity enhancing agent is useful in improvement of skin.

In the case where the OPH activity enhancing agent of the present invention enhances OPH activity in order to degrade the AGE-modified albumin efficiently, the OPH activity enhancing agent preferably contains, for example, at least one kind of plant extract obtained from a plant selected from the group A consisting of water chestnut, stevia, rosemary, thyme, chrysanthemum, savory, and mugwort, more preferably at least one kind of plant extract obtained from a plant selected from the group consisting of water chestnut, stevia, rosemary and thyme. In the case where the OPH activity enhancing agent of the present invention enhances OPH activity in order to degrade the AGE-modified collagen efficiently, the OPH activity enhancing agent preferably contains, for example, at least one kind of plant extract obtained from a plant selected from the group B consisting of chestnut (astringent skin), thyme, savory, rosemary, spearmint, chrysanthemum, water chestnut, and stevia, more preferably at least one kind of plant extract obtained from a plant selected from the group consisting of chestnut (astringent skin), thyme, savory, rosemary, spearmint, and chrysanthemum.

In the case where the OPH activity enhancing agent of the present invention enhances OPH activity in order to degrade the AGE-modified albumin and the AGE-modified collagen efficiently, the OPH activity enhancing agent preferably contains, for example, at least one kind of plant extract obtained from a plant selected from the group A and at least one kind of plant extract obtained from a plant selected from the group B.

In particular, it is preferable that the OPH activity enhancing agent contains at least one kind of plant extract obtained from a plant selected from the group consisting of water chestnut, stevia, rosemary, thyme, and chrysanthemum.

The water chestnut (scientific name: *Trapa japonica*) is a plant belonging to the genus *Trapa* of the family Trapaceae. An extract of the water chestnut may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the water chestnut, the part from which the extract is obtained is not particularly limited, and the extract may be a seed coat extract, for example.

The stevia (scientific name: *Stevia rebaudiana*) is a plant belonging to the genus *Stevia* of the family Asteraceae. An extract of the stevia may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the stevia, the part from which the extract is obtained is not particularly limited, and the extract may be a leaf extract, for example.

The rosemary (scientific name: *Rosmarinus officinalis*) is a plant belonging to the genus *Rosmarinus* of the family Lamiaceae. An extract of the rosemary may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the rosemary, the part from which the extract is obtained is not particularly limited, and the extract may be a leaf extract, for example.

The thyme (scientific name: *Thymus vulgaris*) is a plant belonging to the genus *Thymus* of the family Lamiaceae. An extract of the thyme may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the thyme, the part from which the extract is obtained is not particularly limited, and the extract may be a leaf extract, for example.

The chrysanthemum (scientific name: *Chrysanthemum morifolium*) is a plant belonging to the genus *Chrysanthemum* of the family Asteraceae. An extract of the chrysanthemum may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the chrysanthemum, the part from which the extract is obtained is not particularly limited, and the extract of may be a petal extract, for example. Specific examples of the petal extract include a petal extract obtained from an edible chrysanthemum.

The savory (scientific name: *Satureia hortensis*) is a plant belonging to the genus *Satureja* of the family Lamiaceae. An extract of the savory may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the savory, the part from which the extract is obtained is not particularly limited, and the extract may be a leaf extract or a spike extract, for example.

The mugwort (scientific name: *Artemisa indica*) is a plant belonging to the genus *Artemisia* of the family Asteraceae. An extract of the mugwort may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the mugwort, the part from which the extract is obtained is not particularly limited, and the extract may be a leaf extract, for example.

The chestnut (scientific name: *Castanea crenata*) is a plant belonging to the genus *Castanea* of the family Fagaceae. An extract of the chestnut may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the chestnut, the part from which the extract is obtained is not particularly limited, and the extract may be, for example, an extract obtained from outer skin or astringent skin.

The spearmint (scientific name: *Mentha spicata*) is a plant belonging to the genus *Mentha* of the family Lamiaceae. An extract of the spearmint may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the spearmint, the part from which the extract is obtained is not particularly limited, and the extract may be a leaf extract, for example.

The marjoram (scientific name: *Origanum majorana*) is a plant belonging to the genus *Origanum* of the family Lamiaceae. An extract of the marjoram may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the marjoram, the part from which the extract is obtained is not particularly limited, and the extract may be a leaf extract, for example.

The peppermint (scientific name: *Mentha piperita*) is a plant belonging to the genus *Mentha* of the family Lamiaceae. An extract of the peppermint may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the peppermint, the part from which the extract is obtained is not particularly limited, and the extract may be a leaf extract, for example.

The lemon balm (scientific name: *Melissa officinalis*) is a plant belonging to the genus *Melissa* of the family Lamiaceae. An extract of the lemon balm may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the lemon balm, the part from which the extract is obtained is not particularly limited, and the extract may be a leaf extract, for example.

The allspice (scientific name: *Pimenta dioica*) is a plant belonging to the genus *Pimenta* of the family Myrtaceae. An extract of the allspice may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the allspice, the part from which the extract is obtained is not particularly limited, and the extract may be a fruit extract or a leaf extract, for example.

The perilla (scientific name: *Perilla frutescens*) is a plant belonging to the genus *Perilla* of the family Lamiaceae. An extract of the perilla may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the perilla, the part from which the extract is obtained is not particularly limited, and the extract may be a spike extract, for example. Specific examples of the spike extract include an extract of inflorescence of perilla (young spike of perilla).

The basil (scientific name: *Ocimum basilicum*) is a plant belonging to the genus *Ocimum* of the family Lamiaceae. The basil is a plant often used in, for instance, Italian food etc., and also is known by the name of "basilico", "basil", and "sweet basil". An extract of the basil may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the basil, the part from which the extract is obtained is not particularly limited, and the extract may be a leaf extract, for example.

The caraway (scientific name: *Carum carvi*) is a plant belonging to the genus *Carum* of the family Apiaceae. An extract of the caraway may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant, for example. In the extract of the caraway, the part from which the extract is obtained is not particularly limited, and the extract may be a leaf extract, for example.

In the present invention, the extract can be obtained from any of the above-described parts or from the entire plant, for example. The method for obtaining the extract is not particularly limited, and examples thereof include known methods such as solvent extraction and expression.

An extraction solvent to be used in the solvent extraction is not particularly limited, and examples thereof include aqueous solvents and organic solvents. The aqueous solvents are not particularly limited, and examples thereof include water. The organic solvents are not particularly limited, and examples thereof include: alcohols such as lower alcohols and polyhydric alcohols; ketones; esters; ethers; nitriles; aromatic compounds; and alkyl chlorides. The lower alcohols are not particularly limited, and examples thereof include methanol, ethanol, and absolute ethanol. The polyhydric alcohols are not particularly limited, and examples thereof include propylene glycol and 1,3-butylene glycol. The ketones are not particularly limited, and examples thereof include acetone and formic acid. The esters are not particularly limited, and examples thereof include ethyl acetate. The ethers are not particularly limited, and examples thereof include diethyl ether and dioxane. The nitriles are not particularly limited, and examples thereof include acetonitrile. The aromatic compounds are not particularly limited, and examples thereof include benzene, toluene, and xylene. The alkyl chlorides are not particularly limited, and examples thereof include chloroform.

As the above-described extraction solvent, one kind of solvent may be used, or two or more kinds of solvents may be used in combination, for example. Furthermore, the extraction solvent may be a mixed solvent of the aqueous solvent and the organic solvent, for example. The mixed solvent is not particularly limited, and examples thereof include lower alcohol aqueous solutions such as ethanol aqueous solutions. The proportion of the organic solvent in the mixed solvent is not particularly limited, and is, for example, about 1 vol% to about 99 vol%.

The solvent extraction can be carried out by, for example, providing any of the above-described parts from which an extract is obtained or the entire plant as a raw material and immersing the raw material in the extraction solvent. The raw material may be subjected to treatments such as, for example, washing, drying, and pulverizing, prior to the immersion. When there are two or more kinds of raw materials, each of the raw materials may be subjected to an extraction treatment separately, or a mixture of the two or more kinds of raw materials may be subjected to an extraction treatment. In the former case, for example, the resultant extracts may be mixed together to provide a mixed extract. The proportions of the respective extracts in the mixed extract are not particularly limited, and may be equal proportions (weights), for example. In the latter case, for example, the proportions of the respective raw materials in the mixture are not particularly limited, and may be equal proportions (weights), for example.

The ratio between the raw material and the extraction solvent used in the extraction is not particularly limited. For example, with respect to 100 g (dry weight) of the raw material, there may be 1 to 1000 L or 1 to 100 L of the extraction solvent. The immersion time is not particularly limited, and can be set as appropriate depending on, for example, the kinds, the amounts, and the like of the raw material and the extraction solvent. Specifically, in the case where 100 g of the raw material is immersed in 10 L of the extraction solvent, the immersion time is, for example, about 0.5 hours or longer, or about 0.5 to about 24 hours.

The temperature of the extraction solvent at the time of extraction is not particularly limited, and may be room temperature, equal to or higher than room temperature, or equal to or lower than room temperature. When the extraction solvent is an aqueous solvent, the extraction treatment preferably is hot water extraction, for example. In the case of the hot water extraction, the temperature of the aqueous solvent is not particularly limited, and the lower limit thereof is, for example, 30°C or higher or 50°C or higher, and the temperature range is, for example, from 50°C to 100°C. Furthermore, the treatment time in the hot water extraction is not particularly limited, and can be set as appropriate depending on, for example, the kind and the amount of the raw material, the amount of the aqueous solvent, and the like. Specifically, when extraction from 100 g (dry weight) of the raw material is performed using 10 L of the aqueous solvent, the treatment time may be such that the lower limit thereof is, for example, about 0.5 hours or longer, and the range thereof is, for example, from 0.5 to 24 hours.

After the extraction treatment, the obtained extract may be subjected to a purification treatment or the like, for example. The purification treatment is not particularly limited, and examples thereof include known methods such as a distillation treatment, a filtration treatment, a chromatography treatment, and a drying treatment.

Specific examples of the extraction method include water extraction, alcohol extraction, micellar extraction, and supercritical extraction. By the water extraction, it is possible to obtain an extract with higher safety at a lower cost, for example. By the alcohol extraction, components that cannot be extracted with water can be collected and also can be sterilized, for example. By the micellar extraction, it is possible to extract both components soluble in water and components soluble in alcohols, for example. By the supercritical extraction, it is possible to obtain an extract with higher extraction efficiency.

Examples of the filtration treatment include an ultrafiltration treatment. In the ultrafiltration treatment, the conditions for molecular weight fractionation are not particularly limited, and the molecular weight of a fraction to be collected is, for example, 10 kDa or less.

The form of the extract is not particularly limited. For example, the extract may be in the form of liquid, paste, powder, or the like. The form of the extract can be selected as appropriate depending on the form of the OPH activity enhancing agent to be described below.

The OPH activity enhancing agent may contain only the above-described extract, or it may be a composition containing the above-described extract and other components. Examples of the components include various kinds of additives such as an excipient, a binding agent, a lubricant, a disintegrant, an absorption promoter, an emulsifying agent, a stabilizing agent, and an antiseptic agent. The blended amounts of the various kinds of additives in the OPH activity enhancing agent are not particularly limited, and can be set as appropriate.

The form of the OPH activity enhancing agent is not particularly limited. For example, the OPH activity enhancing agent may be in the form of solid, gel, liquid, or the like. Specific examples of the form of the OPH activity enhancing agent include powder, microgranule, granule, tablet, coated tablet, capsule, troche, eye drop, liquid, patch, lotion, and ointment.

The OPH activity in vivo can be enhanced by administering the OPH activity enhancing agent of the present invention to a subject, for example. Thus, the OPH activity enhancing agent can be used in, for example, a method for treating or preventing diseases, senescence of tissues, hypofunction of tissues, and the like caused by an oxidized protein or a glycated protein such as AGE-modified protein in vivo, as will be described below. The OPH activity enhancing agent may be administered in the form of a composition obtained by mixing it with components of another article selected from, for example, foods such as common foods and nutritional supplements, cosmetics, quasi drugs, and pharmaceuticals. The blended amount of the OPH activity enhancing agent in the composition can be set as appropriate depending on, for example, the kind of the article, a subject to which the composition is administered, and the like, and is not particularly limited. The OPH activity enhancing agent of the present invention also can be referred to as, for example, a therapeutic agent or a preventive agent for diseases, senescence of tissues, hypofunction of tissues, and the like caused by the oxidized protein or the glycated protein in vivo. The pharmaceutical composition according to the present invention is characterized in that it contains the OPH activity enhancing agent of the present invention.

As described above, the method for enhancing OPH activity according to the present invention includes the step of enhancing activity of OPH in a sample by adding the OPH activity enhancing agent of the present invention to the sample containing the OPH. The above description as to the OPH activity enhancing agent of the present invention also applies to the method of the present invention, for example. In the method for enhancing OPH activity according to the present invention, the sample may be a biological sample collected from a subject or may be a non-biological sample, for example.

As described above, the method for accelerating protein degradation according to the present invention includes the step of accelerating degradation of an oxidized protein or a glycated protein in a sample by OPH by adding the OPH activity enhancing agent of the present invention to the sample containing the protein and the OPH. The above description as to the OPH activity enhancing agent of the present invention also applies to the method of the present invention, for example. In the method for accelerating protein degradation according to the present invention, the sample may be a biological sample collected from a subject or may be a non-biological sample, for example.

Examples of the oxidized protein and the glycated protein include: proteins modified by diseases showing pathological conditions due to aging or senescence; proteins suffering oxidative damage by, for example, exposure to ultraviolet rays; and proteins modified by sugar. Examples of the modification of the proteins include oxidation, glycation, carbonylation, and acylation. The carbonylation encompasses conversion to hydroxynonenal, malondialdehyde, glycolaldehyde, and pyrraline, for example. Specifically, examples of the oxidized protein include, but are not limited to, oxidized oxiredoxin, and examples of the glycated protein include, but are not limited to, HbAlc, glycoalbumin, and proteins modified with AGEs (Advanced Glycation End-products).

The treatment method according to the present invention treats diseases, senescence of tissues, and/or hypofunction of tissues caused by the production of an oxidized protein or a glycated protein by administering the OPH activity enhancing agent of the present invention to a subject. The treatment method of the present invention may be a prevention method. Examples of the diseases include diabetes complications, Alzheimer's disease, cataracts, arteriosclerosis, osteoporosis, rheumatism, and osteoarthropathy. Examples of the tissues include skin, nerves, the kidney, blood vessels, the retina, the crystalline lens, bones, joints, brain tissues, the liver, and erythrocytes.

A subject to which the OPH activity enhancing agent of the present invention is administered is not particularly limited, and may be a human or a non-human animal, for example. Examples of the latter include: nonhuman mammals such as monkeys, cows, pigs, dogs, and cats; birds such as chickens; and fish and shellfish. The administration method is not particularly limited, and examples thereof include oral administration and parenteral administration. Examples of the parenteral administration include transdermal administration and injections such as intravenous injection.

The form of the OPH activity enhancing agent is not particularly limited, and may be in any of the above-described forms, for example. Furthermore, the OPH activity enhancing agent may be administered in the form of a composition obtained by mixing it with other components, as described above. The blended amount of the OPH activity enhancing agent in the composition can be set as appropriate, and is not particularly limited, as described above. The dose of the OPH activity enhancing agent can be set as appropriate depending on, for example, the animal species and age, and is not particularly limited. When a healthy adult orally takes the OPH activity enhancing agent, the dose of the OPH activity enhancing agent is as follows: the amount of the extract (solid content) contained therein is such that the lower limit thereof is, for example, 10 mg or 50 mg, the upper limit thereof is 2000 mg or 1000 mg, and the range thereof is, for example, from 10 to 2000 mg or from 50 to 1000 mg. The OPH activity enhancing agent of the present invention also can be referred to as a therapeutic agent for treating the above-described diseases, senescence of tissues, and/or hypofunction of tissues, and it also encompasses a preventive agent, for example.

The extract of the present invention is at least one kind of plant extract obtained from a plant selected from the group consisting of water chestnut, stevia, rosemary, thyme, chrysanthemum, savory, mugwort, chestnut, spearmint, marjoram, peppermint, lemon balm, allspice, perilla, basil, and caraway, for use in treatment of diseases, senescence of tissues, and/or hypofunction of tissues caused by the production of an oxidized protein or a glycated protein. The treatment may be prevention.

The extract of the present invention is at least one kind of plant extract obtained from a plant selected from the group consisting of water chestnut, stevia, rosemary, thyme, chrysanthemum, savory, mugwort, chestnut, spearmint, marjoram, peppermint, lemon balm, allspice, perilla, basil, and caraway, for use in production of the OPH activity enhancing agent or the therapeutic or preventive agent.

The composition according to the present invention is a composition containing at least one kind of plant extract obtained from a plant selected from the group consisting of water chestnut, stevia, rosemary, thyme, chrysanthemum, savory, mugwort, chestnut, spearmint, marjoram, peppermint, lemon balm, allspice, perilla, basil, and caraway, for use in treatment of diseases, senescence of tissues, and hypofunction of tissues caused by the production of an oxidized protein or a glycated protein. The treatment may be prevention. In the extract and the composition of the present invention, the extract of each kind and the composition containing the extract are as described above, and the combination, the method for using them, and the like also are as described above.

The composition of the present invention is a composition containing at least one kind of plant extract obtained from a plant selected from the group consisting of water chestnut, stevia, rosemary, thyme, chrysanthemum, savory, mugwort, chestnut, spearmint, marjoram, peppermint, lemon balm, allspice, perilla, basil, and caraway, for use in production of the OPH activity enhancing agent or the therapeutic or preventive agent.

Examples

Next, examples of the present invention will be described. It is to be noted, however, that the present invention is by no means limited by the following examples.

[Example 1]

In the present example, effects of plant extracts on OPH activity were evaluated with AGE-modified human serum albumin as an index.

(1) Preparation of extract samples of plants

Plants were cut with reference to Shokuhin Seibun Hyou 2012 (Tables of Food Composition 2012, Kagawa Nutrition University Publishing Division). Thus, plant samples of the following plants were obtained. The plant samples were dried at 65°C for 20 hours using a dryer (Taiki Sangyo food dryer Petit Mini, TAIKISANGYO CO., LTD.). The thus-obtained dried samples were turned to powder by pulverization with a pulverizer (Labo Milser LM-PLUS, OSAKA CHEMICAL Co., Ltd.). Then, 2 g of each of the powder samples was suspended in 40 mL of distilled water at 80°C, and subjected to an extraction treatment for 1 hour. Thereafter, the suspension was centrifuged, and the supernatant was obtained. The thus-obtained supernatants were used as extract samples.

Water chestnut: seed coats
Stevia: leaves
Rosemary: leaves
Thyme: leaves
Chrysanthemum (edible chrysanthemum): petals
Savory: leaves and spikes
Mugwort: leaves
Chestnut: outer skins or astringent skins
Spearmint: leaves
Marjoram: leaves
Peppermint: leaves
Lemon balm: leaves
Allspice: fruits and leaves
Perilla: young spikes of perilla
Basil: leaves
Caraway: leaves

(2) Measurement of AGE-degrading activity of OPH

40 mg/mL human serum albumin (code: A-16532, Sigma), 100 mmol/L phosphate buffer solution (pH 7.4), 2 mol/L glucose, and distilled water were mixed together at a volume ratio of 2 : 5 : 1 : 2. The resultant liquid mixture was incubated at 60°C for 40 hours. Thus, AGE-modified human serum albumin (AGE-HSA) was prepared. This was used as a substrate for measurement of degrading activity of OPH.

As OPH, 0.5 U/mL Acylamino-acid-releasing enzyme (AARE, code 7301, TAKARA BIO INC.) was used (the same applies hereinafter). A reaction solution having the following composition was prepared and incubated at 37°C for 90 minutes. After the incubation, 100 $\mu$L of 7% perchloric acid (PCA) aqueous solution was added to the reaction solution, and the resultant mixture was cooled with ice for 20 minutes. Thereafter, the mixture was centrifuged at 4°C, and the precipitate was collected. The precipitate was dissolved in 350 $\mu$L of 200 mmol/L Tris-HCl (pH 7.4). The thus-obtained solution was added to a 96-well black microplate so that each well contained 250 $\mu$L of the solution. Then, AGEs-derived fluorescence (excitation wavelength: 370 nm, detection wavelength: 440 nm) in each of the wells was measured with a microplate reader. At the same time, as a control, a reaction solution not containing any extract sample was prepared, and the fluorescence measurement was carried out in the same manner. Then, each measured value was converted to a relative value, assuming that the fluorescence value in 5 $\mu$g/mL quinine sulfate solution was 1,000, and the enhancement ratio (%) of the AGE-degrading activity of OPH was calculated using the following equation. The AGE-degrading activity enhancement ratio calculated using the following equation is an enhancement ratio of the degrading activity of OPH in the example (where the extract samples were added; indicated as "S" hereinafter) to the degrading activity of OPH in the control (where no extract sample was added; indicated as "C" hereinafter). Enhancement ratio (%) of AGE-degrading activity of OPH = [1 - (S/C)] $\times$ 100

# EP 2 957 289 A1

[Table 1]

(Composition of Reaction Solution)

| | Example (S) | Control (C) |
|---|---|---|
| | (µL) | (µL) |
| 200 mmol/L Tris-HCl (pH 7.4) | 110 | 120 |
| AGE-HSA | 120 | 120 |
| OPH (0.5 U/mL) | 10 | 10 |
| Extract sample | 10 | 0 |

The results thereof are shown in Table 2 below. As can be seen from Table 2 below, in the example where the extract samples were added, higher OPH activity enhancements were observed as compared with the control. In particularly, the activity enhancement by the water chestnut extract was very high. These results demonstrate that the respective plant extracts can enhance the OPH activity. Therefore, it can be said that they can accelerate the degradation and removal of AGE-modified proteins.

[Table 2]

| Plant | Activity enhancement ratio (%) |
|---|---|
| Water chestnut | 48.49 |
| Stevia | 29.98 |
| Rosemary | 29.83 |
| Thyme | 25.61 |
| Chrysanthemum | 21.92 |
| Savory | 18.62 |
| Mugwort | 18.15 |
| Chestnut (outer skins) | 16.66 |
| Spearmint | 15.80 |
| Chestnut (astringent skins) | 14.72 |
| Marjoram | 13.73 |
| Peppermint | 13.09 |
| Lemon balm | 10.05 |
| Allspice | 9.88 |
| Perilla | 9.71 |
| Basil | 9.54 |
| Caraway | 9.35 |

[Example 2]

In the present example, effects of ultrafiltered plant extracts on OPH activity were evaluated with AGE-modified human serum albumin as an index.

(1) Preparation of extract samples of plants

Dried samples of the following plants were provided, and they were subjected to an extraction treatment in the same manner as in Example 1. Thereafter, regarding each sample, the supernatant was collected by centrifugation. Then, the supernatant was ultrafiltered using an ultrafiltration filter with a molecular weight cut-off of 10 kDa (trade name: Amicon Ultra-0.5 Centrifugal Filter Unit with Ultracel-10 membrane, Merck KGaA (Merck Millipore Division)), and a low molecular weight fraction with a molecular weight of less than 10 kDa was collected. The thus-collected low molecular weight fractions were used as extract samples.

Water chestnut: seed coats
Stevia: leaves
Rosemary: leaves
Thyme: leaves

Chrysanthemum (edible chrysanthemum): petals
Savory: leaves and spikes
Spearmint: leaves

(2) Measurement of AGE-degrading activity of OPH

The AGE-modified human serum albumin (AGE-HSA) used in Example 1 was used as a substrate for measurement of degrading activity of OPH. Also, four kinds of reaction solutions (A to D) having the following compositions were prepared (n = 3). In each of the reaction solutions, the respective components were added in the following order: Tris-HCl, the extract sample, OPH, and AGE-HSA.

[Table 3]

|  | Reaction solution (μL) | | | |
|---|---|---|---|---|
|  | A | B | C | D |
| 200 mmol/L Tris-HCl (pH 7.4) | 150 | 160 | 240 | 240 |
| AGE-HAS | 80 | 80 | 0 | 0 |
| OPH (0.5 U/mL) | 10 | 10 | 10 | 0 |
| Extract sample | 10 | 0 | 0 | 10 |

After each reaction solution was prepared by mixing the components, the reaction solution was incubated at 37°C for 18 hours. After the incubation, 100 μL of 7% perchloric acid (PCA) aqueous solution was added to the reaction solution, and the resultant mixture was cooled with ice for 20 minutes. Thereafter, the mixture was centrifuged at 4°C, and the precipitate was collected. The precipitate was dissolved in 350 μL of 200 mmol/L Tris-HCl (pH 7.4). The thus-obtained solution was added to a 96-well black microplate so that each well contained 200 μL of the solution. Then, AGEs-derived fluorescence (excitation wavelength: 370 nm, detection wavelength: 440 nm) in each of the wells was measured with a microplate reader. Each measured value was converted to a relative value, assuming that the fluorescence value in 5 μg/mL quinine sulfate solution was 1,000, and the enhancement ratio (%) of the AGE-degrading activity of OPH was calculated using the following equation.

$$\text{Enhancement ratio (\%) of AGE-degrading activity of OPH}$$
$$= [1 - (A - C - D)/(B - C)] \times 100$$

A: Relative value obtained regarding reaction solution A
B: Relative value obtained regarding reaction solution B
C: Relative value obtained regarding reaction solution C
D: Relative value obtained regarding reaction solution D

The results thereof are shown in Table 4 below. As can be seen from Table 4 below, in the example where the samples collected as the low molecular weight fraction by the ultrafiltration were added, higher OPH activity enhancements were observed as compared with the control. In particularly, the activity enhancement by the thyme was very high. These results demonstrate that the OPH activity can be enhanced by the respective plant extracts. Therefore, it can be said that they can accelerate the degradation and removal of AGE-modified proteins.

[Table 4]

| Plant | Activity enhancement ratio (%) |
|---|---|
| Thyme | 48.49 |
| Savory | 38.71 |
| Rosemary | 35.38 |
| Spearmint | 32.93 |
| Chrysanthemum | 32.85 |
| Water Chestnut | 29.41 |

(continued)

| Plant | Activity enhancement ratio (%) |
|---|---|
| Stevia | 17.68 |

[Example 3]

In the present example, effects of plant extracts on OPH activity were evaluated with AGE-modified collagen as an index.

(1) Preparation of extract samples of plants

Dried samples of the following plants were provided, and they were subjected to an extraction treatment in the same manner as in Example 1. Thus, extract samples were prepared.

Water chestnut: seed coats
Stevia: leaves
Rosemary: leaves
Thyme: leaves
Chrysanthemum (edible chrysanthemum): petals
Savory: leaves and spikes
Mugwort: leaves
Chestnut: outer skins or astringent skins
Spearmint: leaves
Marjoram: leaves
Peppermint: leaves
Lemon balm: leaves

(2) Measurement of AGE-degrading activity of OPH

AGE-modified collagen was prepared using collagen (Type I collagen derived from bovine corium (pepsin solubilized collagen), Nippi, Incorporated). This AGE-modified collagen was used as a substrate for measurement of degrading activity of OPH. The measurement of the AGE-degrading activity was carried out in the same manner as in Example 1, except that the collagen was used instead of the human serum albumin and that the incubation time was set to 10 days instead of the 40 hours in the preparation of the AGE-modified collagen.

The results thereof are shown in Table 5 below. As can be seen from Table 5 below, in the example where the extract samples were added, higher OPH activity enhancements were observed as compared with the control. In particularly, the activity enhancements by the rosemary extract and the chestnut (astringent skins) extract were very high. These results demonstrate that the respective plant extracts can enhance the OPH activity. Therefore, it can be said that they can accelerate the degradation and removal of AGE-modified proteins.

[Table 5]

| Plant | Activity enhancement ratio (%) |
|---|---|
| Chestnut (astringent skins) | 98.82 |
| Rosemary | 81.59 |
| Thyme | 74.33 |
| Lemon balm | 69.70 |
| Water chestnut | 69.53 |
| Savory | 65.09 |
| Spearmint | 62.48 |
| Chestnut (outer skins) | 49.56 |
| Chrysanthemum | 47.88 |
| Marjoram | 46.25 |
| Peppermint | 39.88 |
| Stevia | 29.15 |
| Mugwort | 22.16 |

While the present invention has been described above with reference to illustrative embodiments, the present invention is by no means limited thereto. Various changes and modifications that may become apparent to those skilled in the art may be made in the configuration and specifics of the present invention without departing from the scope of the present invention.

This application claims priority from Japanese Patent Application No. 2013-029339 filed on February 18, 2013. The entire disclosure of this Japanese Patent Application is incorporated herein by reference.

Industrial Applicability

According to the present invention, it is possible to enhance OPH activity. Thus, the present invention can be used to accelerate the degradation of damaged proteins such as oxidized proteins and glycated proteins. Therefore, the present invention also can be used to treat or prevent diseases, senescence of tissues, and hypofunction of tissues caused by the damaged proteins produced in vivo. The present invention shows excellent safety because it uses plant extracts that have been used since ancient times. Also, the present invention shows excellent productivity because the plant extracts can be obtained in large quantities. The present invention is applicable to a wide range of fields such as industrial products, foods, and pharmaceuticals.

**Claims**

1. An oxidized protein hydrolase activity enhancing agent comprising:

   at least one kind of plant extract obtained from a plant selected from the group consisting of water chestnut, stevia, rosemary, thyme, chrysanthemum, savory, mugwort, chestnut, spearmint, marjoram, peppermint, lemon balm, allspice, perilla, basil, and caraway.

2. The oxidized protein hydrolase activity enhancing agent according to claim 1, wherein the oxidized protein hydrolase activity enhancing agent comprises two or more kinds of the plant extracts.

3. A method for enhancing activity of oxidized protein hydrolase, the method comprising the step of:

   enhancing activity of oxidized protein hydrolase in a sample by adding the oxidized protein hydrolase activity enhancing agent according to claim 1 or 2 to the sample.

4. A method for accelerating protein degradation, the method comprising the step of:

   accelerating degradation of an oxidized protein or a glycated protein in a sample by oxidized protein hydrolase by adding the oxidized protein hydrolase activity enhancing agent according to claim 1 or 2 to the sample.

5. A pharmaceutical composition comprising:

   the oxidized protein hydrolase activity enhancing agent according to claim 1 or 2.

## INTERNATIONAL SEARCH REPORT

| International application No. |
| --- |
| PCT/JP2014/053474 |

**A. CLASSIFICATION OF SUBJECT MATTER**
*A61K36/00*(2006.01)i, *A61K36/18*(2006.01)i, *A61K36/28*(2006.01)i,
*A61K36/53*(2006.01)i, *A61P9/10*(2006.01)i, *A61P19/02*(2006.01)i,
*A61P19/10*(2006.01)i, *A61P25/28*(2006.01)i, *A61P27/12*(2006.01)i,
According to International Patent Classification (IPC) or to both national classification and IPC

**B. FIELDS SEARCHED**

Minimum documentation searched (classification system followed by classification symbols)
A61K36/00, A61K36/18, A61K36/28, A61K36/53, A61P9/10, A61P19/02,
A61P19/10, A61P25/28, A61P27/12, A61P29/00, A61P43/00

Documentation searched other than minimum documentation to the extent that such documents are included in the fields searched
Jitsuyo Shinan Koho          1922–1996    Jitsuyo Shinan Toroku Koho    1996–2014
Kokai Jitsuyo Shinan Koho    1971–2014    Toroku Jitsuyo Shinan Koho    1994–2014

Electronic data base consulted during the international search (name of data base and, where practicable, search terms used)
CAplus/MEDLINE/EMBASE/BIOSIS(STN), JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2010-77123 A (Hayashikane Sangyo Co., Ltd.), 08 April 2010 (08.04.2010), claims; paragraphs [0001] to [0011], [0024]; examples (Family: none) | 1,2,5 |
| X | JP 2003-63976 A (Ichimaru Pharcos Co., Ltd.), 05 March 2003 (05.03.2003), claims; paragraphs [0008], [0019] to [0020]; examples (Family: none) | 1,2,5 |
| X | JP 2005-281221 A (Taisho Pharmaceutical Co., Ltd.), 13 October 2005 (13.10.2005), claims; examples (Family: none) | 1,5 |

[X] Further documents are listed in the continuation of Box C.      [ ] See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 07 May, 2014 (07.05.14) | 20 May, 2014 (20.05.14) |

| Name and mailing address of the ISA/ Japanese Patent Office | Authorized officer |
| --- | --- |
| Facsimile No. | Telephone No. |

Form PCT/ISA/210 (second sheet) (July 2009)

**INTERNATIONAL SEARCH REPORT**

| International application No. |
| --- |
| PCT/JP2014/053474 |

C (Continuation).    DOCUMENTS CONSIDERED TO BE RELEVANT

| Category* | Citation of document, with indication, where appropriate, of the relevant passages | Relevant to claim No. |
| --- | --- | --- |
| X | JP 5-306214 A  (Mikimoto Pharmaceutical Co., Ltd.),<br>19 November 1993 (19.11.1993),<br>claims; paragraphs [0002], [0010]; examples<br>& US 5538731 A | 1,2,5 |
| E,X | JP 2014-37399 A  (Up Well Co., Ltd.),<br>27 February 2014 (27.02.2014),<br>claims; paragraphs [0001] to [0014], [0023] to [0030], [0040]; examples<br>(Family: none) | 1,2,5 |

Form PCT/ISA/210 (continuation of second sheet) (July 2009)

**INTERNATIONAL SEARCH REPORT**

International application No.

PCT/JP2014/053474

Continuation of A. CLASSIFICATION OF SUBJECT MATTER
  (International Patent Classification (IPC))

*A61P29/00*(2006.01)i, *A61P43/00*(2006.01)i

        (According to International Patent Classification (IPC) or to both national
        classification and IPC)

Form PCT/ISA/210 (extra sheet) (July 2009)

**INTERNATIONAL SEARCH REPORT**

International application No.

PCT/JP2014/053474

| Box No. II | Observations where certain claims were found unsearchable (Continuation of item 2 of first sheet) |
|---|---|

This international search report has not been established in respect of certain claims under Article 17(2)(a) for the following reasons:

1. ☒ Claims Nos.: 3, 4
because they relate to subject matter not required to be searched by this Authority, namely:
Claims 3 and 4 pertain to "method for treatment of the human body or animal body by surgery or therapy" and thus relate to a subject matter on which this International Searching Authority is not required to carry out an international search under the provisions of PCT Article 17(2)(a)(i) and [PCT Rule 39.1(iv)].

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:
In the inventions according to claims 1, 2 and 5, multiple plant extracts that are stated as alternatives are each used as "an oxidized protein hydrolase activity enhancer". It appears that the concept common to these invention that are stated as alternatives is "an oxidized protein hydrolase activity enhancer" that contains some plant extract.
As disclosed in WO 2011/004733 A1, however, using a plant extract as "an oxidized protein hydrolase activity enhancer" is a technique that had been known prior to the filing of the present application and, therefore, does not make a contribution over the prior art.
(Continued to extra sheet)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
In the inventions according to claims 1, 2 and 5, those relating to a *Trapa japonica* extract.

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2014/053474

Continuation of Box No.III of continuation of first sheet(2)

Therefore, it cannot be considered that the above-said technique is a special technical feature of invention.

Such being the case, claims 1, 2 and 5 include 16 invention groups in total, namely, the inventions relating to "oxidized protein hydrolase activity enhancers" respectively containing 16 kinds of the plant extract.

Form PCT/ISA/210 (extra sheet) (July 2009)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002179592 A
- JP 4255432 B
- JP 2008308505 A
- JP 2004091398 A
- JP 2007099650 A
- WO 2011004733 A
- JP 2013029339 A

**Non-patent literature cited in the description**

- **GOTO et al.** *Experimental Medicine,* 1998, vol. 18, 2324-2331
- **BREUSING N. et al.** *Biol. Chem.,* 2008, vol. 389, 203-209
- Tables of Food Composition. Kagawa Nutrition University Publishing Division, 2012